# EUROPEAN PATENT APPLICATION

(11) **EP 4 389 193 A1**
(43) Date of publication of application: **26.06.2024**
(21) Application number: 22215009.6
(22) Date of filing: 20.12.2022
(51) Int. Cl.: A61N 1/05, A61B 5/00, A61B 8/00, A61N 1/36, A61N 1/375, A61N 1/372, A61N 7/00, B06B 1/00

(54) **AN APPARATUS, A SYSTEM AND A METHOD FOR STIMULATING A PART OF A PERIPHERAL NERVOUS SYSTEM**

(71) Applicant: Stichting IMEC Nederland, 5656 AE Eindhoven (NL)
(72) Inventor: Beutel, Fabian, 40547 Düsseldorf (DE)
(74) Representative: AWA Sweden AB

(57) **Abstract**

An apparatus (100) for stimulating a part of a peripheral nervous system comprises: a sensor unit (110) comprising at least one ultrasound transducer (112; 612) configured to transmit ultrasound into the part of the peripheral nervous system and at least one pair of electrodes (114; 614) configured to detect electrical signals in the part of the peripheral nervous system, wherein the sensor unit (110) is configured to determine structural information and functional information of the part of the peripheral nervous system; a stimulation unit (120) configured to transmit a stimulation signal for selective stimulation of a position within the part of the peripheral nervous system; and a controller (140) configured to receive the structural information and the functional information and configured to control a location of the selective stimulation within the part of the peripheral nervous system based on the structural information and the functional information.

## Description

### Technical field

The present description relates to an apparatus and a system for stimulating a part of a peripheral nervous system. The present description also relates to a method for controlling stimulation of the part of the peripheral nervous system.

### Background

Bioelectric medicine (BEM) relates to treating diseases, such as chronic diseases, by electrically interacting with the nervous system of a patient. In general, BEM typically refers to interaction with the peripheral nervous system (PNS), which comprises any neural structure outside of the central nervous system (CNS), consisting of brain and spinal cord.

Targeted BEM opposes conventional pharmaceutical drugs, which are less location-specific and known to cause various side effects. Therefore, targeted BEM is considered highly promising for personalized therapeutic applications. These applications are ranging from neurological disorders like epilepsy and depression, to cardiovascular diseases, autoimmune diseases, and impaired motor function, as well as diabetes treatment via metabolic regulation at organ innervation points.

However, in order to provide efficient results of targeted BEM, there is a need to control electrical interaction with the peripheral nervous system and ensure that a desired effect is provided.

### Summary

An objective of the present description is to provide accurate control of stimulation of a nerve such that an accurate control of a desired effect of stimulation is provided.

This and other objectives are at least partly met by the invention as defined in the independent claims. Preferred embodiments are set out in the dependent claims.

According to a first aspect, there is provided an apparatus for stimulating a part of a peripheral nervous system, said apparatus comprising: a sensor unit comprising at least one ultrasound transducer and at least one pair of electrodes configured to be arranged in different locations in relation to the part of the peripheral nervous system, wherein the at least one ultrasound transducer is configured to transmit ultrasound into the part of the peripheral nervous system and wherein the at least one pair of electrodes is configured to detect electrical signals in the part of the peripheral nervous system, wherein the sensor unit is configured to determine structural information of the part of the peripheral nervous system and functional information of the part of the peripheral nervous system based on at least one sensing mode of the sensor unit, wherein the sensor unit is configured to, in the at least one sensing mode, perform ultrasound detection and/or electrical signal detection based on transmitting ultrasound into the part of the peripheral nervous system; a stimulation unit configured to transmit a stimulation signal into the part of the peripheral nervous system for selective stimulation of a position within the part of the peripheral nervous system; and a controller configured to receive the structural information and the functional information and configured to control a location of the selective stimulation of the position within the part of the peripheral nervous system based on the structural information and the functional information.

Thanks to the first aspect, a multi-modal apparatus is provided to provide a functionality enabling accurate control of stimulation. The apparatus may be configured to sense information of the peripheral nervous system and provide closed-loop stimulation that may be controlled based on the sensed information.

Thanks to the apparatus, it is possible to accurately determine properties of the part of the peripheral nervous system such that a location in which selective stimulation is to be provided may be determined accurately. The stimulation may thus be limited to a particular location associated with a desired function such that the stimulation may provide isolated effect on a particular function in a body of a living being that is to be treated, hereinafter also referred to as a subject. This implies that the location may be very specifically selected such that stimulation only affects particular neural signals.

For instance, a nerve may comprise a plurality of fascicles. Within the nerve, a large number of fascicles may be provided, and each fascicle may include a large number of axons, such that the nerve may transmit signals to / from various parts of the body. For instance, the vagus nerve is a cranial nerve providing signals between the brain and parts of the body. The vagus nerve may be relatively easy to access, and the apparatus may therefore be advantageously arranged in relation to the vagus nerve for providing stimulation of the vagus nerve. However, since the vagus nerve provides signals to many different parts of the body, it is also important that stimulation of the nerve is controlled to selectively stimulate a position of the nerve for providing a desired effect of the stimulation.

Thanks to the apparatus, structural information and functional information of the part of the peripheral nervous system is determined. Thus, a structure of a cross-section of a nerve may be determined and also functionality associated with the structure of the nerve may be determined. This implies that the apparatus is configured to acquire information of where fascicles are arranged within the nerve and information of the neural activity within the fascicles, such that an accurate location to be stimulated for achieving a desired effect may be acquired. Further, the apparatus comprises a stimulation unit that is configured to provide selective stimulation of a position within the part of the peripheral nervous system such that the stimulation unit may be controlled to selectively stimulate the desired location.

It should be realized that in addition to controlling a location of stimulation, the controller may also be configured to control other characteristics of stimulation such as intensity of stimulation signals. Such control may also be based on the structural information and the functional information.

The stimulation of the part of the peripheral nervous system may provide an effect on the peripheral nervous system. The stimulation may for instance modulate a neural signal propagating in the part of the peripheral nervous system. The stimulation may be configured to activate the part of the peripheral nervous system such that a neural signal is transmitted in response to the stimulation. The stimulation may be configured to inhibit a neural signal, e.g., by blocking a neural signal from passing the part of the peripheral nervous system.

The term "*part of the peripheral nervous system*" may refer to a nerve but may not necessarily be a nerve. Rather, the part of the peripheral nervous system may be any part configured to transmit neural signals and may for instance be a ganglion. A "nerve" should be construed as a bundle of nerve fibers enclosed by a sheath called the epineurium. The nerve may form part of the peripheral nervous system and may therefore also be called a peripheral nerve. The nerve is configured to transmit electrical impulses within the body along axons, which may be bundled in fascicles in the nerve. The electrical impulses may be provided as action potentials transmitted along the axons. The apparatus may further be configured to stimulate a part of the peripheral nervous system such that the apparatus may be configured to stimulate a section of the peripheral nervous system, e.g., a section along a longitudinal extension of a nerve. The apparatus may thus be configured to be arranged in relation to the section to be stimulated in order to provide stimulation of the part of the peripheral nervous system.

The sensor unit comprises at least one ultrasound transducer. The sensor unit may advantageously comprise a plurality of ultrasound transducers, which may facilitate more accurate sensing of the structural and/or functional information. However, in some embodiments, a single ultrasound transducer may be sufficient. The ultrasound transducers of the plurality of ultrasound transducers may be arranged in different locations in relation to the part of the peripheral nervous system to facilitate providing spatial selectivity of transmitting and/or detecting ultrasound.

The sensor unit comprises at least one pair of electrodes. The sensor unit may advantageously comprise a plurality of electrodes, such as a set of electrodes, which may allow defining a plurality of pairs of electrodes for providing electrical signal detection. This may facilitate more accurate sensing of the structural and/or functional information. However, in some embodiments, a single pair of electrodes may be sufficient. The electrodes of the plurality of electrodes may be arranged in different locations in relation to the part of the peripheral nervous system to facilitate providing spatial selectivity of electrical signal detection. The pair of electrodes and the transducer may also be arranged in different locations in relation to the part of the peripheral nervous system for providing spatial information relating to the part of the peripheral nervous system.

It should be realized that the at least one ultrasound transducer and at least one pair of electrodes may be arranged relative each other in various ways for being arranged in different locations in relation to the part of the peripheral nervous system. For instance, the at least one ultrasound transducer and at least one pair of electrodes may be arranged in different locations in relation to a common cross-section of a nerve. Alternatively, or additionally, the at least one ultrasound transducer and the at least one pair of electrodes may be arranged in different locations along a longitudinal extension of a nerve. It should be realized that the at least one ultrasound transducer and the pair of electrodes may be arranged in close relation to each other while still being arranged in different locations.

The apparatus may be configured to be implanted in a body of the subject. The sensor unit may be configured to be arranged such that the at least one ultrasound transducer and the at least one pair of electrodes are arranged implanted in the body in close vicinity or in contact with an outer surface of the part of the peripheral nervous system.

The sensor unit may be configured to be set into a plurality of sensing modes. The sensor unit may utilize the plurality of sensing modes in order to determine structural information and functional information of the part of the peripheral nervous system.

The sensor unit may be configured to use the at least one ultrasound transducer and the at least one pair of electrodes in various ways for determining the structural and functional information. It should also be realized that the sensor unit may further comprise other sensing modalities that may complement information acquired by the at least one transducer and the at least one pair of electrodes for determining the structural and functional information.

The sensor unit may be configured to, in one sensing mode, transmit ultrasound into the part of the peripheral nervous system. The sensor unit may further be configured to detect ultrasound and/or electrical signals based on the ultrasound being transmitted into the part of the peripheral nervous system.

The at least one sensing mode may include acoustic sensing based on performing ultrasound detection, such as pulse-echo imaging. The at least one sensing mode may further include acousto-electric sensing, wherein ultrasound transmitted into the part of the peripheral causes an acousto-electric interaction which may further be modulated by neural activity, and wherein the modulated acousto-electric interaction may be detected by electrical signal detection by the at least one pair of electrodes.

The at least one sensing mode may further include electrical sensing, such as electrical impedance tomography based on sensing impedance changes in the part of the peripheral nervous system by the electrode. Thus, the at least one sensing mode may include sensing modes which, in addition to sensing based on transmitting ultrasound into the part of the peripheral nervous system, do not involve transmitting of ultrasound. Also, the at least one sensing mode may include further additional sensing modes which do not necessarily involve detection of ultrasound or electrical signals.

The structural information may provide information of structure, such as internal structure, of the part of the peripheral nervous system, based on the sensor unit being arranged externally to the part of the peripheral nervous system. The structural information may for instance provide shape, size, and location of structures within the part of the peripheral nervous system. The structural information may for instance provide information where fascicles and neurons are arranged in a nerve.

The functional information may provide information relating to neural activity in the part of the peripheral nervous system. The functional information may be associated with the structural information such that the information relating to neural activity may be related to a location within the part of the peripheral nervous system.

The functional information may for instance provide indication of whether neural activity in a particular location is afferent (directed towards the brain) or efferent (directed towards a body part, e.g., an organ). The functional information may also provide indication of a destination of neural signals and/or velocity of neural signals. The functional information may be used for characterizing particular parts of the part of the peripheral nervous system, such as nerve sub-bundles, which may be identified in the structural information.

The at least one ultrasound transducer may, in one sensing mode, be configured to transmit a continuous ultrasound wave into the part of the peripheral nervous system. The at least one ultrasound transducer may alternatively or additionally, in another sensing mode, be configured to transmit ultrasound pulses into the part of the peripheral nervous system.

A same ultrasound transducer may be used for transmitting ultrasound into the part of the peripheral nervous system and for performing ultrasound detection. However, it should be realized that the sensor unit may comprise a plurality of ultrasound transducers such that, in one sensing mode, ultrasound is transmitted into the part of the peripheral nervous system by one or more ultrasound transducers and ultrasound detection is performed by other ultrasound transducer(s).

The stimulation unit may be configured to transmit a stimulation signal using any appropriate stimulation element. Thus, the stimulation unit may be configured to use one or more modalities for providing the stimulation signal. The stimulation unit may be configured to transmit a stimulation signal using ultrasound or using an electrical signal.

Thus, in embodiments, the stimulation unit may be configured to output the stimulation signal using ultrasound transducer(s) and/or electrode(s). The stimulation unit may be configured to utilize the ultrasound transducer(s) and/or electrode(s) of the sensor unit to transmit a generated stimulation signal into the part of the peripheral nervous system via such ultrasound transducer(s) and/or electrode(s). However, the stimulation unit may advantageously include stimulation elements separate from sensor elements of the sensor unit.

According to an embodiment, the sensor unit is configured to determine structural information of the part of the peripheral nervous system based on a first sensing mode and determine functional information of the part of the peripheral nervous system based on a second sensing mode.

Thus, the sensor unit may be configured to use different sensing modes for determining structural information and for determining functional information. This implies that each sensing mode may be adapted to acquisition of the information of interest.

It should be realized that the sensor unit may be configured to use more than two sensing modes such that each of the structural information and the functional information may be determined based on one or more sensing modes. Also, some sensing mode(s) may acquire information relevant for both determining structural information and functional information.

According to an embodiment, the controller is configured to receive the structural information and configured to control a selective sensing by the sensor unit of functional information of the part of the peripheral nervous system for determining functional information in selected locations of the part of the peripheral nervous system.

This implies that the functional information need not be generally acquired for an entire part of the peripheral nervous system. Rather, the structural information may define regions within the part of the peripheral nervous system, such as within a cross-section of the nerve, in which neural activity of interest is taking place. Thus, the controller may be configured to control the sensor unit such that functional information need only be acquired from selected regions of the part of the peripheral nervous system.

This implies that efficiency in acquiring the functional information may be vastly increased.

According to an embodiment, the sensor unit is configured to determine functional information of the part of the peripheral nervous system based on an acousto-electric sensing mode, wherein the at least one ultrasound transducer is configured to transmit ultrasound pulses into the part of the peripheral nervous system and the at least one pair of electrodes is configured to detect acousto-electric interaction between the ultrasound pulses and the part of the peripheral nervous system, wherein the acousto-electric interaction is modulated by neural activity.

Thus, the at least one sensing mode may include an acousto-electric sensing mode, which may be useful for determining functional information of the part of the peripheral nervous system.

The neural activity may define an electrical current density within the part of the peripheral nervous system. This electrical current density may modulate the acousto-electric interaction within the part of the peripheral nervous system, such that the neural activity may be detected by the at least one pair of electrodes. However, it should be realized that the acousto-electric interaction may also or alternatively be modulated by other electrical properties associated with the neural activity.

According to an embodiment, the stimulation unit comprises a plurality of stimulation elements configured to be arranged in relation to the part of the peripheral nervous system, wherein the stimulation elements comprise ultrasound transducers and/or electrodes for providing the selective stimulation of the part of the peripheral nervous system by an ultrasound stimulation signal and/or an electrical stimulation signal.

This implies that the stimulation unit may use at least ultrasound stimulation and/or electrical stimulation. This may facilitate providing stimulation such that a location within the part of the peripheral nervous system may be selectively stimulated.

The use of a plurality of stimulation elements may allow the stimulation to be defined based on interference between a plurality of signals transmitted by the plurality of stimulation elements. This facilitates providing selective stimulation (deep) within the part of the peripheral nervous system without providing stimulation close to the stimulation elements.

According to an embodiment, the sensor unit and the stimulation unit are arranged in a carrier configured to conform to an outer surface of the part of the peripheral nervous system.

Thus, the ultrasound transducer(s) and electrodes of the sensor unit and the stimulation elements of the stimulation unit may be arranged in close relation to or in contact with the outer surface of the part of the peripheral nervous system. The carrier may be used for providing a well-defined relation of the ultrasound transducer(s) and electrodes of the sensor unit and the stimulation elements of the stimulation unit to each other and to the part of the peripheral nervous system. The carrier may also facilitate arrangement of the sensor unit and the stimulation unit in relation to the part of the peripheral nervous system, such that implantation may be relatively quickly performed.

It should be realized that the sensor unit and the stimulation unit need not necessarily be arranged in a common carrier. Rather, the apparatus may comprise separate carriers in which the sensor unit and the stimulation unit are arranged.

The apparatus may further comprise drive electronics for the stimulation unit and readout electronics for the sensor unit. The drive electronic and the readout electronics may be part of the stimulation unit and the sensor unit, respectively, or may alternatively form part of the controller.

The controller may also be configured to be arranged in the carrier. Alternatively, the controller may be separately arranged from the sensor unit and the stimulation unit. Also, the controller need not necessarily be arranged in close vicinity to the part of the peripheral nervous system. However, the controller is configured to communicate with the sensor unit and the stimulation unit. In order to ensure a minimal delay in receiving information from the sensor unit by the controller and in transmitting control signals to the stimulation unit, the controller should be configured to be connected to the sensor unit and the stimulation unit through a wired connection.

According to an embodiment, the carrier has form of a cuff configured to be arranged around a nerve.

This may be particularly suitable for providing a robust arrangement of the sensor unit and the stimulation unit in relation to the nerve.

However, it should be realized that other form factors of the carrier may be used. The carrier may for instance have a flexible structure which may be configured to be arranged along a longitudinal direction of the part of the peripheral nervous system without necessarily being arranged entirely surrounding a cross-section of the part of the peripheral nervous system. For instance, the carrier may be in form of a patch. This may also be useful for arranging the carrier in relation to neural innervation points on sub-organ level.

According to an embodiment, the sensor unit comprises a set of ultrasound transducers and a set of electrodes, wherein the set of ultrasound transducers and the set of electrodes are arranged in a sensing area of the carrier with the ultrasound transducers arranged intermixed with electrodes such that at least one ultrasound transducer is arranged between two electrodes.

This may be used for accurately associating ultrasound detection and electrical signal detection with positions within the part of the peripheral nervous system. For instance, both the ultrasound transducers and the electrodes may be distributed around a circumference of a nerve.

The ultrasound transducers and the electrodes may be alternatingly arranged. For instance, the ultrasound transducers and the electrodes may be alternatingly arranged around the circumference of the nerve. This may facilitate providing accurate ultrasound detection and electrical signal detection within an entire cross-section of the nerve.

However, it should be realized that the ultrasound transducers and the electrodes may be arranged intermixed in another manner, which may not necessarily define a regular pattern of ultrasound transducers and electrodes. Also, the number of ultrasound transducers and the number of electrodes need not necessarily be same.

According to an embodiment, the sensor unit comprises a first set and a second set of ultrasound transducers and a first set and a second set of electrodes, wherein the first set of ultrasound transducers and the first set of electrodes are arranged in a first sensing area of the carrier and the second set of ultrasound transducers and the second set of electrodes are arranged in a second sensing area of the carrier, and wherein the first sensing area and the second sensing area are arranged on opposite sides of a stimulation area of the carrier.

The stimulation area may be arranged in relation to a cross-section of the part of the peripheral nervous system. The stimulation elements of the stimulation unit may be arranged in the stimulation area of the carrier.

The first sensing area may be arranged in relation to a first cross-section of the part of the peripheral nervous system and the second sensing area may be arranged in relation to a second cross-section of the part of the peripheral nervous system. The first sensing area, the stimulation area and the second sensing area may be arranged along a direction of propagation of neural signals in the part of the peripheral nervous system, such that the first sensing area and the second sensing area are on opposite sides of the stimulation area along the direction of propagation of neural signals.

This implies that the first set of ultrasound transducers and the first set of electrodes may be configured to detect effects of stimulation by the stimulation unit in a first direction of propagation in the part of the peripheral nervous system and the second set of ultrasound transducers and the second set of electrodes may be configured to detect effects of stimulation by the stimulation unit in a second direction of propagation in the part of the peripheral nervous system, opposite to the first direction. Thus, by use of the first sensing area and the second sensing area on opposite sides of the stimulation area, the sensor unit may easily determine whether neural activity in a particular location of the part of the peripheral nervous system is afferent or efferent.

According to an embodiment, the carrier comprises an acoustically transparent material such that the carrier is configured to be arranged in relation to the part of the peripheral nervous system with the acoustically transparent material between the part of the peripheral nervous system and ultrasound transducers.

This may facilitate providing a focus of an ultrasound beam at a location close to the outer surface of the part of the peripheral nervous system.

The acoustically transparent material may also provide acoustic impedance matching. Alternatively, the carrier comprises an acoustic impedance matching material such that the carrier is configured to be arranged in relation to the part of the peripheral nervous system with the acoustic impedance matching material between the part of the peripheral nervous system and ultrasound transducers, or the carrier may comprise two material layers providing an acoustically transparent material and an acoustic impedance matching material, respectively.

According to an embodiment, the controller is further configured to control one or more of a size of the location of the selective stimulation of the part of the peripheral nervous system, a stimulation modality of the stimulation signal, by the stimulation signal on the part of the peripheral nervous system, and timing of the stimulation signal.

Thus, the controller may be configured to accurately control the stimulation.

The control of the stimulation modality may provide control whether the stimulation should involve an ultrasound stimulation signal and/or an electrical stimulation signal or another type of stimulation signal.

The control of the effect by the stimulation signal may provide control whether the stimulation should cause activation of a neural signal in the part of the peripheral nervous system, whether the stimulation should cause modulation of amplitude, phase and/or frequency of a neural signal propagating in the part of the peripheral nervous system or whether the stimulation should inhibit a neural signal in the part of the peripheral nervous system.

According to a second aspect, there is provided a system for stimulating a part of a peripheral nervous system, said system comprising: the apparatus according to the first aspect; and a biomarker sensor configured to sense information of a biomarker of a part of a body.

Effects and features of this second aspect are largely analogous to those described above in connection with the first aspect. Embodiments mentioned in relation to the second aspect are largely compatible with the first aspect.

Thanks to using a biomarker sensor, the biomarker may be monitored.

The apparatus may be intended to be used for providing stimulation in order to affect a particular part of the body in a desired manner. Thus, by using a biomarker sensor, the biomarker may provide input whether the desired control is provided by the stimulation of the part of the peripheral nervous system by the apparatus.

The apparatus may be arranged in relation to the part of the peripheral nervous system being remote from the part of the body to be affected. Thus, the biomarker sensor may be arranged separately from the apparatus.

The biomarker sensor may be configured to be arranged externally to the body. Thus, the biomarker sensor may not need to be implanted. In some embodiments, the biomarker sensor may be configured to be attached to skin of the subject.

According to an embodiment, the system comprises a systemic controller configured to receive the information of the biomarker and configured to monitor effect of the selective stimulation of the position within the part of the peripheral nervous system by the stimulation unit of the apparatus.

Thus, the effect of the selective stimulation by the apparatus may be monitored to ensure that a desired effect is achieved. The systemic controller may be configured to provide an output when a desired effect is not achieved. This may be used by a user for checking whether the apparatus may need to be controlled.

According to an embodiment, the systemic controller is configured to provide input to the controller of the apparatus based on the information of the biomarker for controlling the selective stimulation of the position within the part of the peripheral nervous system by the stimulation unit of the apparatus.

This implies that the controller of the apparatus may receive input based on the biomarker and may control the stimulation in dependence of the biomarker.

Thus, the system may be self-controlling for providing adaption of the stimulation in dependence of the effect of stimulation at a part of the body.

The systemic controller may be arranged in a common housing with the biomarker sensor. Alternatively, the systemic controller may be arranged in a separate housing.

Both the systemic controller and the biomarker sensor may be arranged externally to the subject. This may facilitate communication between the systemic controller and the biomarker sensor. The communication may be through wired or wireless communication. The systemic controller may be configured for wireless communication with the controller of the apparatus. This facilitates arranging the systemic controller externally to the subject with the apparatus being implanted in the subject.

According to a third aspect, there is provided a method for controlling stimulation of a part of a peripheral nervous system, said method comprising: determining structural information of the part of the peripheral nervous system; determining functional information of the part of the peripheral nervous system; and determining a location within the part of the peripheral nervous system for which selective stimulation is desired, wherein the location of the selective stimulation within the part of the peripheral nervous system is determined based on the structural information and the functional information.

Effects and features of this third aspect are largely analogous to those described above in connection with the first and second aspects. Embodiments mentioned in relation to the third aspect are largely compatible with the first and second aspects.

The method allows accurately determining a location within the part of the peripheral nervous system to which stimulation is to be applied. Thanks to the method being able to provide structural and functional information of the part of the peripheral nervous system, the stimulation may be selectively determined to a particular location that is associated with a desired function.

The method may further comprise sending a control signal to a stimulation unit. The control signal may be configured to provide parameters to the stimulation unit such that the stimulation unit may be able to output a stimulation signal to provide selective stimulation to the desired location.

It should be realized that the method for controlling the stimulation signal may be performed in any controller, which is able to provide control signals for controlling the stimulation unit. Thus, the method does not involve steps for outputting the stimulation signal, but rather only involves the steps for controlling the stimulation signal to be output. This control may be provided by a controller within the apparatus for stimulating a part of the peripheral nervous system. The method relates to an ingenious manner of using detailed information relating to a part of a peripheral nervous system for enabling determining a location to which a stimulation signal is to be output. However, the method does not relate to the output of the stimulation signal.

According to a fourth aspect, there is provided a method for controlling stimulation of a part of a peripheral nervous system, said method comprising: determining structural information of the part of the peripheral nervous system; determining functional information of the part of the peripheral nervous system; and transmitting a stimulation signal into the part of the peripheral nervous system for selective stimulation of a position within the part of the peripheral nervous system, wherein a location of the selective stimulation of the position within the part of the peripheral nervous system is controlled based on the structural information and the functional information.

Effects and features of this fourth aspect are largely analogous to those described above in connection with the first, second, and third aspects. Embodiments mentioned in relation to the fourth aspect are largely compatible with the first, second, and third aspects.

The method allows accurate control of stimulation of the part of the peripheral nervous system. The method may provide selected stimulation such that an effect of the stimulation may address a particular desired effect while ensuring that undesired side effects are avoided or at least limited.

### Brief description of the drawings

The above, as well as additional objects, features, and advantages of the present inventive concept, will be better understood through the following illustrative and non-limiting detailed description, with reference to the appended drawings. In the drawings like reference numerals will be used for like elements unless stated otherwise.
Fig. 1 is a schematic view of a system according to an embodiment.
Fig. 2 is a flow chart of a method according to an embodiment.
Fig. 3 is a flow chart of another method according to another embodiment.
Fig. 4 is a flow chart of control of stimulation according to an embodiment.
Fig. 5 is a schematic view illustrating different arrangements of ultrasound transducers and electrodes.

### Detailed description

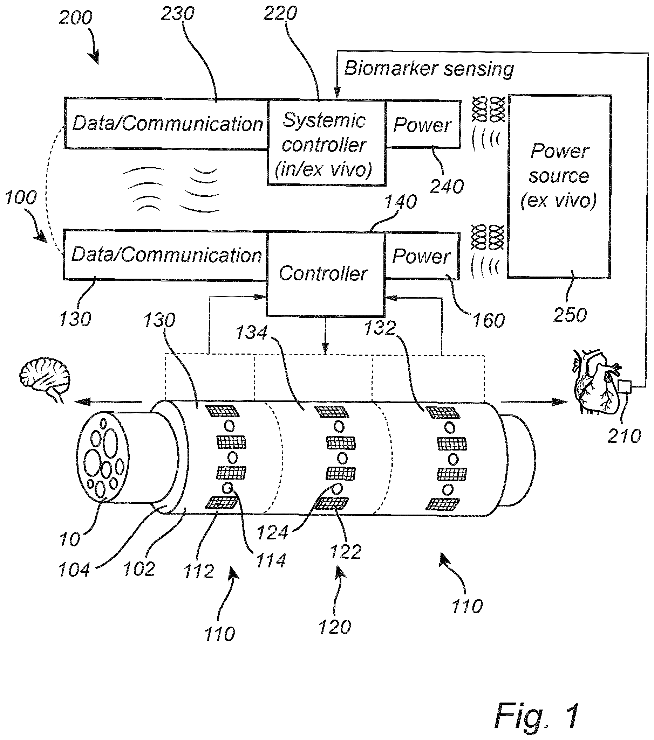

Referring now to Fig. 1, an apparatus 100 for stimulating a part of a peripheral nervous system and a system 200 including the apparatus will be described. The apparatus 100 is shown in Fig. 1 having a cuff 102 arranged around a nerve 10. It should be realized that the part of the peripheral nervous system need not necessarily be a nerve but in the following description, for brevity, reference will be done to a nerve.

The apparatus 100 provides a multi-modal function in that the apparatus 100 may comprise at least ultrasound transducers and electrodes for sensing and/or stimulation of neural activity. The apparatus 100 may be configured to provide sensing and/or stimulation in plural sites along a longitudinal direction of the nerve 10. As shown in Fig. 1, a cuff 102 may be configured to be arranged (implanted) to extend along the longitudinal direction of the nerve 10. The cuff 102 may include several areas for sensing and/or stimulation. However, it should be realized that the apparatus 100 may alternatively comprise a plurality of cuffs and that more or less sites for sensing and/or stimulation may be used.

The apparatus 100 may comprise a sensor unit 110 configured to sense information relating to structural information of the nerve 10 and functional information of the nerve 10. The sensor unit 110 comprises at least one ultrasound transducer. In Fig. 1, small arrays of ultrasound transducers 112 are shown in a plurality of locations in relation to the nerve 10. The ultrasound transducers 112 in an array may be used in combination for forming an ultrasound beam, for instance for focusing the ultrasound beam using phased delays. However, it should be realized that individual ultrasound transducers 112 may instead be arranged separate from each other in different locations. The sensor unit 110 further comprises at least one pair of electrodes, in Fig. 1 shown as a plurality of electrodes 114.

The ultrasound transducers 112 and electrodes 114 are arranged in different locations in relation to the nerve 10 so as to be spatially distributed in relation to the nerve 10. The ultrasound transducers 112 and the electrodes 114 may thus be configured to sense information of the nerve 10 with a spatial resolution to provide accurate and high resolution information relating to the nerve 10.

The ultrasound transducers 112 are configured to transmit ultrasound into the nerve 10. This may be used for detecting information relating to the nerve 10 based on interaction between the ultrasound and the nerve 10. The ultrasound transducers 112 may further be configured to perform ultrasound detection to detect an ultrasound response based on transmitting the ultrasound into the nerve 10. The same ultrasound transducers 112 may be used for transmitting and detecting ultrasound. Alternatively, ultrasound transducers 112 may be specifically dedicated for transmitting ultrasound or for detecting ultrasound.

The electrodes 114 are configured to detect electrical signals in the nerve 10. The electrodes 114 may be configured to electrical signals based on acousto-electric interaction caused by ultrasound being transmitted into the nerve 10. The electrodes 114 may alternatively be configured to detect electrical signals in the nerve 10, which may or may not involve providing an electrical signal into the nerve 10.

The sensor unit 110 may be configured to be used in at least one sensing mode but may preferably be set into a plurality of sensing modes. Thus, information may be acquired by the sensor unit in at least one sensing mode but may preferably be acquired in a plurality of sensing modes. The sensor unit 110 may be configured to determine structural information of the nerve 10 and functional information of the nerve 10.

The structural information may provide information of structure, such as internal structure, of the nerve 10. The structural information may for instance provide shape, size, and location of structures within the nerve 10. The structural information may for instance provide information where fascicles and neurons are arranged in the nerve 10.

The functional information may provide information relating to neural activity in the nerve 10. The functional information may be associated with the structural information such that the information relating to neural activity may be related to a location within the nerve.

The functional information may for instance provide indication of whether neural activity in a particular location is afferent (directed towards the brain) or efferent (directed towards a body part, e.g., an organ). The functional information may also provide indication of a destination of neural signals, such as to which organ or other part of the body the neural signal is destined, and/or velocity of neural signals. The functional information may be used for characterizing particular parts of the nerve 10, such as nerve sub-bundles, which may be identified in the structural information.

The apparatus 100 further comprises a stimulation unit 120 configured to transmit a stimulation signal into the nerve 10. The stimulation unit 120 may be configured to provide selective stimulation of a position within the nerve 10. This implies that the stimulation unit 120 may be able to focus a stimulation signal or form a stimulation signal in a particular position without affecting the nerve 10 around the particular position.

The stimulation unit 120 may, as shown in Fig. 1, comprise a plurality of stimulation elements. This implies that the stimulation elements may transmit a plurality of signals so as to cause a stimulation signal within the nerve 10 based on interference of individual signals. The stimulation unit 120 may in such manner be configured to selectively stimulate a position within the nerve 10.

The stimulation unit 120 may comprise a plurality of ultrasound transducers 122 and a plurality of electrodes 124. This implies that the stimulation unit 120 may be configured to provide stimulation by an ultrasound stimulation signal and/or an electrical stimulation signal. The stimulation unit 120 may be configured to be controlled to select whether an ultrasound stimulation signal or an electrical stimulation signal is to be used so as to select a modality used for stimulation.

The cuff 102 may comprise an acoustically transparent material 104 such that at least part of the acoustically transparent material 104 may be arranged between the nerve 10 and the ultrasound transducers 112, 122 of the sensor unit 110 and the stimulation unit 120. This may be used for ensuring that an ultrasound beam transmitted by the ultrasound transducers 112, 122 may be focused in an area within the nerve 10 close to the outer surface.

The apparatus 100 may comprise ultrasound transducers 112, 122 as part both of the sensor unit 110 and the stimulation unit 120. Using a same type of ultrasound transducers 112, 122 for both the sensor unit 110 and the stimulation unit 120 may simplify manufacturing of the apparatus 100. However, the type of ultrasound transducer to be used may be selected based on functionality requirements, such that different types of ultrasound transducers may be used for the sensor unit 110 and the stimulation unit 120, or even for different ultrasound transducers in the sensor unit 110 depending on whether the ultrasound transducer is to be used for transmitting or detecting ultrasound, or both.

The ultrasound transducers 112, 122 may for instance be conventional ultrasound transducers based on a piezoelectric material, such as lead zirconate titanate (PZT), capacitive micromachined ultrasound transducers (cMUT) or piezoelectric micromachined ultrasound transducers (pMUT).

The apparatus 100 further comprises a controller 140. The controller 140 may be configured to receive information from the sensor unit 110 such that the controller 140 may be provided with access to structural information and functional information of the nerve 10. The controller 140 may further be configured to control the stimulation unit 120 such that the stimulation by the stimulation unit may be steered so as to provide selective stimulation in a desired location based on the structural information and the functional information. The controller 140 may further use other information for controlling the stimulation to be provided by the stimulation unit 140.

The controller 140 may be configured to not only control a location of selective stimulation. Rather, the controller 140 may be configured to control one or more of a size of the location of the selective stimulation of the nerve 10, a stimulation modality of the stimulation signal, a type of effect by the stimulation signal on the nerve 10, and timing of the stimulation signal.

The sensor unit 110, the stimulation unit 120, and the controller 140 may all be arranged in a common housing, such as the cuff 102. For illustration purposes, the controller 140 is illustrated outside the cuff 102 in Fig. 1, but it should be realized that the controller 140 may further be arranged in the cuff 102. The sensor unit 110 may comprise circuitry for acquiring measurement signal and possibly (pre-)processing the measurement signal, such as providing analog-to-digital conversion of the measurement signal. The stimulation unit 120 may also comprise circuitry for generating a stimulation signal based on input parameters.

The controller 140 may be implemented in a general-purpose processing unit, such as a central processing unit (CPU), which may execute instructions of one or more computer programs in order to implement functionality of the controller 140. However, the controller 140 may alternatively be implemented as firmware arranged e.g., in an embedded system, or as a specifically designed processing unit, such as an Application-Specific Integrated Circuit (ASIC) or a Field-Programmable Gate Array (FPGA).

Thus, the sensor unit 110, the stimulation unit 120 and the controller 140 may be implemented as separate units within the cuff 102 and may be configured to communicate by transmitting signals between the units within the cuff 102. However, it should be realized that functions of the sensor unit 110, the stimulation unit 120 and the controller 140 may even be intertwined, such that parts of the sensor unit 110 and the stimulation unit 120 may also be implemented in a common processor also forming the controller 140 so that (parts of) the different units may be defined by different threads within the common processor.

Further, it should be realized that the sensor unit 110, the stimulation unit 120 and the controller 140 may be arranged in separate physical housings. Whereas the sensor unit 110 and the stimulation unit 120 may need to be arranged in close relation to the nerve 10, the controller 140 may be remotely arranged. The sensor unit 110, the stimulation unit 120 and the controller 140 may be configured to communicate through wired communication between different housings.

The apparatus 100 may define a first sensing site 130 and a second sensing site 132 on opposite sides of a stimulation site 134. This implies that the apparatus 100 may be configured to sense neural activity on opposite sides of a stimulation cross-section of the nerve 10 in which stimulation occurs, to detect neural activity in relation to propagation of neural signals in an afferent or efferent direction in relation to the stimulation cross-section.

The sensor unit 110 may comprise a first set and a first set of electrodes associated with the first sensing site 130. The sensor unit 110 may further comprise a second set of ultrasound transducers a second set of electrodes associated with the second sensing site 134.

Each sensing site and the stimulation site may comprise alternating ultrasound transducers and electrodes circumferentially arranged around the nerve 10.

The sensor unit 110 may be configured to perform structural acoustic sensing at high resolution. The sensor unit 110 may for instance be configured to perform pulse-echo imaging for ultrasound detection based on ultrasound pulses being transmitted into the nerve 10. This may be used for determining structural information of the nerve 10 with high resolution.

The sensor unit 110 may further be configured to perform further acoustic or electrical signal detections for determining structural information of the nerve 10. Such further detections may be used as contrasting mechanisms distinguishing material characteristics of the nerve 10.

The sensor unit 110 may be configured to perform elastography using ultrasound detection or electrical impedance tomography (EIT) using electrical signal detection. The EIT may be used for determining structural information and functional information of the nerve 10.

The sensor unit 110 may further be configured to perform acousto-electric sensing, wherein the sensor unit 110 is configured to determine functional information of the nerve 10 at high resolution. The acousto-electric sensing may be performed by transmitting ultrasound pulses into the nerve 10 and detecting acousto-electric interaction between the ultrasound pulses and the nerve 10, wherein the acousto-electric interaction is modulated by neural activity.

The functional information may be acquired by actively providing a signal into the nerve 10. The signal may be focused at a location of the nerve 10 and the focused signal may further be moved within the nerve 10 in order to acquire information relating to different locations.

According to an embodiment, the structural information may be used for controlling determining of functional information. Thus, the functional information may not need to be acquired for each location within the nerve 10. The sensor unit 110 may thus first be used for determining structural information based on a first sensing mode and may then be used for determining functional information based on a second sensing mode.

The controller 140 may be configured to receive the structural information. The controller 140 may further be configured to control the sensor unit 110 such that the functional information may be acquired by selective sensing in selected locations in the nerve 10. This implies that an active signal may be steered only to the specific locations for which it is desired to acquire functional information.

For instance, when the sensor unit 110 is configured to acquire functional information using acousto-electric sensing, beam steering of ultrasound may be used for controlling the ultrasound beam to be focused at selected locations, whereby functional information may be acquired from the selected locations. This implies that a speed of acquiring the functional information of interest may be vastly improved.

The apparatus 100 may further comprise a communication unit 150 configured to communicate with an external unit. The apparatus 100 may be configured to be implanted in a subject and the communication unit 150 may be configured to communicate information to an external unit outside the subject, such as information relating to the nerve 10 or information relating to control of stimulation. The external unit may be configured to analyze the information received from the apparatus 100 and may provide information for updating control of stimulation by the apparatus 100.

The communication unit 150 may be configured to communicate with the external unit using wireless communication. The communication unit 150 may be connected to the controller 140 so as to provide communication between the controller 140 and the external unit.

The apparatus 100 may further comprise a power source 160. The power source 160 may be configured to be (re-)charged through wireless power transfer to allow the power source 160 to be (re-)charged while implanted in the subject.

It should be realized that the apparatus 100 described above may operate in isolation and control of stimulation may be solely based on the controller 140 having access to structural and functional information of the nerve 10. However, as shown in Fig. 1, the system 200 may be configured to combine the apparatus 100 with further components which may be arranged externally to the subject.

The system 200 may comprise a biomarker sensor 210 which is configured to sense information of a biomarker of a part of a body. The biomarker sensor 210 may thus sense information relating to the part of the body which may be desired to affect by the stimulation provided by the apparatus 100.

The biomarker sensor 210 may be any type of sensor suitable for acquiring information of characteristics of a part of a body. For instance, the biomarker sensor 210 may be configured to sense heart rate, blood pressure, motion, metabolic compound concentration, etc. A frequency at which biomarker information is sensed and controlled may be application-specific and may range from single to a few Hz for heart rate up to kHz for motor function.

The biomarker sensor 210 may be connected to a systemic controller 220, which may be configured to receive the sensed information of the biomarker from the biomarker sensor 210. The systemic controller 220 may be configured to monitor an effect of the stimulation of the nerve 10 on the biomarker.

The systemic controller 220 may be implemented in a general-purpose processing unit, such as a central processing unit (CPU), which may execute instructions of one or more computer programs in order to implement functionality of the systemic controller 220. However, the systemic controller 220 may alternatively be implemented as firmware arranged e.g., in an embedded system, or as a specifically designed processing unit, such as an Application-Specific Integrated Circuit (ASIC) or a Field-Programmable Gate Array (FPGA).

The systemic controller 220 may be configured to communicate with the apparatus 100. The systemic controller 220 may thus be associated with a communication unit 230 configured to communicate wirelessly with the communication unit 150 of the apparatus 100. The systemic controller 220 may provide input to the controller 140 of the apparatus 100 for controlling the stimulation by the apparatus 100. The systemic controller 220 may provide control signals for controlling stimulation in view of the biomarker information or may alternatively transmit biomarker information such that the controller 140 of the apparatus 100 may use the biomarker information in controlling the stimulation.

The biomarker sensor 210 and the systemic controller 220 may be configured to be arranged in one or more housings which may be worn by the subject. The systemic controller 220 may be associated with a power source 240 for providing power to the systemic controller 220. This power source 240 may be provided as a battery which may be configured to be (re-)charged by an external power source 250 through wired or wireless power transfer.

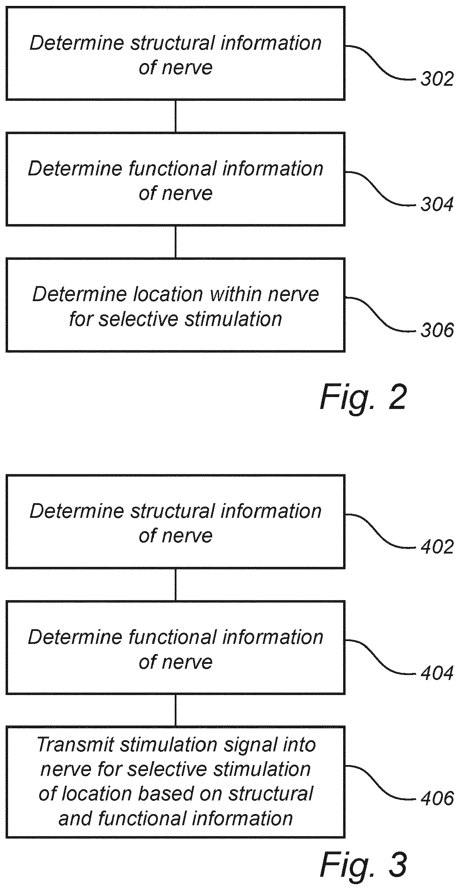

Referring now to Fig. 2, a method for controlling stimulation of the nerve 10 will be briefly summarized.

The method comprises determining 302 structural information of the nerve and determining 304 functional information of the nerve 10. The structural information and the functional information of the nerve 10 may be determined based on measurements performed using the at least one ultrasound transducer 112 and the at least one pair of electrodes 114 of the sensor unit 110. The sensor unit 110 may thus obtain information based on measurements. The determining of the structural information and the functional information may be performed by processing results of the measurements. Thus, the steps of determining of structural information and functional information may involve processing of results of measurements but the processing does not involve actually acquiring the results of measurements.

The method further comprises determining 306 a location within the nerve 10 for which selective stimulation is desired, wherein the location of the selective stimulation within the nerve 10 is determined based on the structural information and the functional information.

The method allows accurately determining a location within the nerve 10 to which stimulation is to be applied.

The method may further comprise sending a control signal to the stimulation unit 120. The control signal may be configured to provide parameters to the stimulation unit 120 such that the stimulation unit may be able to output a stimulation signal to provide selective stimulation to the desired location.

The method may be performed within one or more processing units, such as within the controller 140. The method is thus a computer-implemented method.

Referring now to Fig. 3, a method for stimulation of the nerve 10 will be briefly summarized.

The method comprises determining 402 structural information of the nerve 10 and determining 404 functional information of the nerve 10. The structural information and the functional information of the nerve 10 may be determined based on measurements performed using the at least one ultrasound transducer 112 and the at least one pair of electrodes 114 of the sensor unit 110. The sensor unit 110 may thus obtain information based on measurements.

The method allows accurately determining a location within the nerve 10 to which stimulation is to be applied based on the structural and functional information.

The method further comprises transmitting 406 a stimulation signal into the nerve 10 for selective stimulation of a position within the nerve 10, wherein a location of the selective stimulation of the position within the nerve 10 is controlled based on the structural information and the functional information.

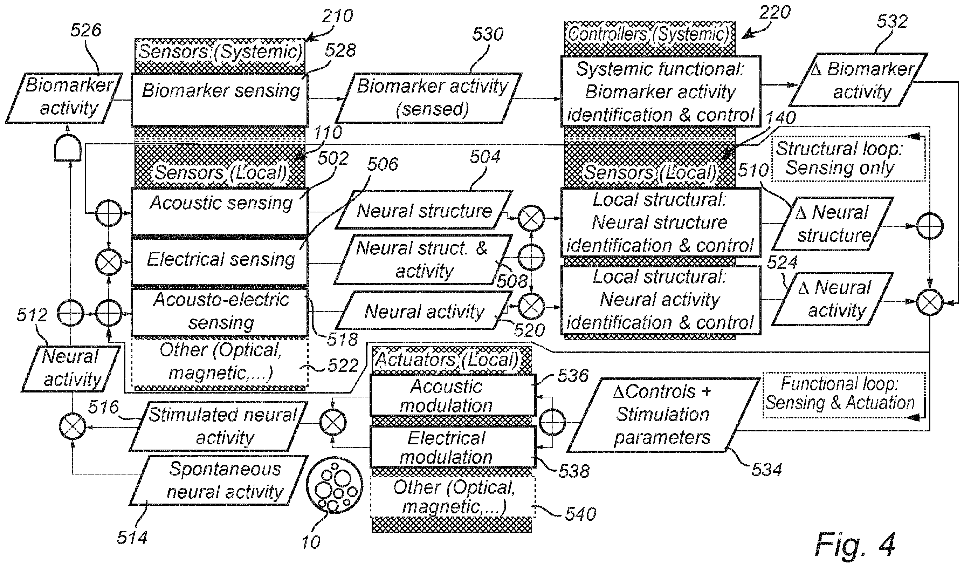

Referring now to Fig. 4, stimulation of the nerve 10 will be described in more detail.

The stimulation of the nerve 10 may be seen as a closed loop where input is based on sensed neural activity associating structural and functional information of the nerve 10 (and possibly also based on biomarker sensing) and output is targeted selective stimulation in response to the input.

First, the acquiring of input involves a structural loop. The structural loop is configured to provide a structural geometrical frame for subsequent sensing and stimulation.

The structural loop may comprise acoustic sensing 502, such as pulse-echo imaging, to generate information 504 relating to structure of the nerve 10. The structural loop may further comprise electrical signal sensing 506, such as EIT sensing, to generate information 508 relating to structure of the nerve 10. It should be realized that these sensing modes may also involve acquiring some functional information in addition to structural information.

The structural information may be provided to the controller 140, which may combine information 504, 508 generated by both the acoustic sensing 502 and the electrical signal sensing 506.

Thus, information on the neural structure, such as position and dimensions of nerve sub-bundles, from acoustic and/or electrical signal sensing may be provided to the controller 140, which is configured to store the structural information.

The structural information may be sporadically controlled to validate whether the apparatus 100 remains in position without translational or rotational changes with respect to the nerve 10. As indicated in Fig. 4, the controller 140 may thus examine whether there is a difference 510 in neural structure in relation to the apparatus 100, which may thus trigger returning to sensing 502 and 504 for validating the structural information.

The acquiring of input also involves a functional loop. Based on the structural information, the sensing of neural activity in the functional loop can be targeted at specific parts of the nerve.

Neural activity 512 may take place as spontaneous neural activity 514 and based on stimulation forming stimulated neural activity 516. The neural activity may be detected based on the electrical signal sensing 504, such as EIT sensing, to generate information 508 relating also to function of the nerve 10.

The neural activity may in particular be detected based on acousto-electric sensing 518, wherein an acoustic beam is focused at locations in the nerve 10 to cause acousto-electric interaction between the nerve 10 and an ultrasound beam. The ultrasound beam may be controlled based on the structural information such that functional information may be acquired only in regions of interest of the nerve 10. The acousto-electric sensing 518 may thus further generate information 520 relating to function of the nerve 10.

The selective sensing of the neural activity may be based on spontaneous neural activity but may also be based on stimulated neural activity such that the determining of functional information may also involve a control by the controller 140 to cause stimulation by the stimulation unit 120 to generate stimulated neural activity.

It should further be realized that the sensor unit 110 may be configured to perform additional sensing relating to the nerve 10 based on other types of sensing modalities such as optical or magnetic sensing 522. This may be used as additional input relating to structural information and/or functional information of the nerve 10.

The functional information may be provided to the controller 140, which may combine information 508, 520 generated by both the electrical signal sensing 506 and the acousto-electric sensing 518.

Thus, information on the neural function, such as direction and velocity of neural signals in sub-bundles of the nerve 10, from electrical signal and/or acousto-electric sensing may be provided to the controller 140, which is configured to store the functional information.

Once functional information is known, the controller 140 may be configured to receive information on sensed neural activity, e.g., from the electrical signal sensing 506 and the acousto-electric sensing 518, and the controller 140 may be configured to determine a difference 524 between the sensed neural activity and an application-specific reference value or a desired neural activity.

A frequency at which neural activity is sensed and controlled may range up to 20 kHz in order to acquire (compound) action potentials of neural signals at millisecond accuracy and to facilitate assessment of velocity of conduction of neural signals.

The neural activity 512 may cause an effect on a biomarker, forming biomarker activity 526. The control of stimulation may further involve biomarker sensing 528 in order to sense the biomarker activity using the biomarker sensor 210. A type of sensor used for biomarker sensing may depend on a property to be sensed.

The biomarker sensing 528 may generate information 530 of sensed biomarker activity 520, which may be provided as input to the systemic controller 220.

The optional systemic controller 220 may thus receive application-specific biomarker activity (e.g., heart rate, blood pressure, motion, metabolic compound concentration, etc.), whose modulation by means of neuromodulation is the ultimate target. The frequency at which the biomarker activity is sensed and controlled is also application-specific and may range from single Hz for heart rate up to kHz for motor function.

The systemic controller 220 and the controller 140 of the apparatus 100 may generate controls based on differences 524, 532, between the sensed information and application-specific reference values or desired neural activity or effect of neural activity. Thus, delta values in neural and biomarker activity may be determined by the systemic controller 220 and the controller 140 of the apparatus 100.

The delta values together with the structural information may be used for determining a set of stimulation parameters 534 to achieve application-specific desired level of neural activity or effect of neural activity. These parameters comprise, but are not limited to, target location and size, the stimulation modality and type of effect (i.e., activating or inhibiting neural activity), as well as specific response delay, intensity, and duration.

The stimulation by the stimulation unit 120 may be based on focused acoustic beams 536, electrical pulses 538 or temporal interference stimulation of either modality (acoustic or electrical). Additionally, or alternatively, the stimulation may be based on another modality 540, such as using optical or magnetic stimulation.

In turn, the sensor unit 110, the biomarker sensor 210, the controller 140 and the systemic controller 220 may also facilitate a function to validate the neural response of the applied stimulation.

Application-specific reference values of the neural and biomarker activity, as well as corresponding stimulation schemes to target these values may come (partly as pre-set parameters) on board with the controller 140 and/or be identified on an individual basis. Identification of application-specific reference values may be facilitated by an automated system identification feature of the controller 140 or systemic controller 220 for the respective control loop. That is, an identification of the neural and/or biomarker response characteristics in terms of magnitude and phase, either to spontaneous neural activity or a sweep in modulation parameters, e.g., frequency, may be performed. In addition, the controller 140 and/or the systemic controller 220 may feature an interface for calibration, customization and finetuning of stimulation parameters, which interface may be accessed by a medical professional or device specialist.

The closed loop control of stimulation may operate at high frequencies to quickly adapt and finetune the stimulation schemes based on the sensed neural and biomarker activity.

It should be realized that the sensor unit 110 and the stimulation unit 120 may be arranged in alternative manners to what is shown in Fig. 1. Alternative dimensions or arrangement of elements of the sensor unit 110 and the stimulation unit 120 may be considered for technical reasons, such as to avoid cross-talk between elements, or for adapting to specific orientations of peripheral neural anatomy.

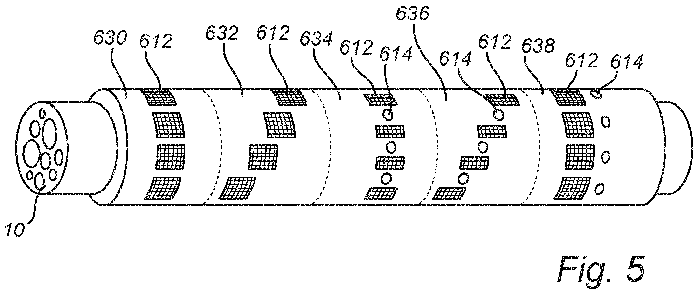

Referring now to Fig. 5, different layouts of the sensor unit 110 and the stimulation unit 120 will be discussed. It should be realized that Fig. 5 illustrates different layouts within a single cuff and that each of the different layouts of the sensor unit 110 and the stimulation unit 120 may be combined with any other layout. In particular, the sensor unit 110 may likely have an identical layout in the first sensing site and the second sensing site but the layout in the first sensing site and the second sensing site need not necessarily be identical.

Fig. 5 shows layouts of elements in five different sites. Any of these layouts may be used for the sensor unit 110 or the stimulation unit 120.

In a first site 630, Fig. 5 shows a single acoustic modality comprising ultrasound transducers 612 of the sensing unit 110 or the stimulation unit 120 which may be arranged in a circumferential arrangement around a single cross-section of the nerve 10.

In a second site 632, Fig. 5 shows a single acoustic modality along an extension of the nerve 10 comprising ultrasound transducers 612 of the sensing unit 110 or the stimulation unit 120 which may be arranged in a helical arrangement extending along the longitudinal direction of the nerve 10.

In a third site 634, Fig. 5 shows combined acoustic and electric modalities comprising ultrasound transducers 612 and electrodes 614 of the sensing unit 110 or the stimulation unit 120 which may be alternatingly arranged in a circumferential arrangement around a single cross-section of the nerve 10.

In a fourth site 636, Fig. 5 shows combined acoustic and electric modalities along an extension of the nerve 10 comprising ultrasound transducers 612 and electrodes 614 of the sensing unit 110 or the stimulation unit 120 which may be alternatingly arranged in a helical arrangement extending along the longitudinal direction of the nerve 10.

In a fifth site 638, Fig. 5 shows combined acoustic and electric modalities comprising ultrasound transducers 612 and electrodes 614 of the sensing unit 110 or the stimulation unit 120, wherein the ultrasound transducers 612 are arranged in a circumferential arrangement around a first cross-section of the nerve 10 and the electrodes 614 are arranged in a circumferential arrangement around a second cross-section of the nerve 10, wherein the first and second cross-sections are close to each other. The ultrasound transducers 612 may be arranged sufficiently close to each other so as to enable focusing of ultrasound into the second cross-section surrounded by electrodes 614.

In the above the inventive concept has mainly been described with reference to a limited number of examples. However, as is readily appreciated by a person skilled in the art, other examples than the ones disclosed above are equally possible within the scope of the inventive concept, as defined by the appended claims.

## Claims

1. An apparatus (100) for stimulating a part of a peripheral nervous system, said apparatus (100) comprising:
a sensor unit (110) comprising at least one ultrasound transducer (112; 612) and at least one pair of electrodes (114; 614) configured to be arranged in different locations in relation to the part of the peripheral nervous system, wherein the at least one ultrasound transducer (112; 612) is configured to transmit ultrasound into the part of the peripheral nervous system and wherein the at least one pair of electrodes (114; 614) is configured to detect electrical signals in the part of the peripheral nervous system, wherein the sensor unit (110) is configured to determine structural information of the part of the peripheral nervous system and functional information of the part of the peripheral nervous system based on at least one sensing mode of the sensor unit (110), wherein the sensor unit (110) is configured to, in the at least one sensing mode, perform ultrasound detection and/or electrical signal detection based on transmitting ultrasound into the part of the peripheral nervous system;
a stimulation unit (120) configured to transmit a stimulation signal into the part of the peripheral nervous system for selective stimulation of a position within the part of the peripheral nervous system; and
a controller (140) configured to receive the structural information and the functional information and configured to control a location of the selective stimulation of the position within the part of the peripheral nervous system based on the structural information and the functional information.

2. The apparatus according to claim 1, wherein the sensor unit (110) is configured to determine structural information of the part of the peripheral nervous system based on a first sensing mode and determine functional information of the part of the peripheral nervous system based on a second sensing mode.

3. The apparatus according to claim 2, wherein the controller (140) is configured to receive the structural information and configured to control a selective sensing by the sensor unit (110) of functional information of the part of the peripheral nervous system for determining functional information in selected locations of the part of the peripheral nervous system.

4. The apparatus according to any one of the preceding claims, wherein the sensor unit (110) is configured to determine functional information of the part of the peripheral nervous system based on an acousto-electric sensing mode, wherein the at least one ultrasound transducer (112; 612) is configured to transmit ultrasound pulses into the part of the peripheral nervous system and the at least one pair of electrodes (114; 614) is configured to detect acousto-electric interaction between the ultrasound pulses and the part of the peripheral nervous system, wherein the acousto-electric interaction is modulated by neural activity.

5. The apparatus according to any one of the preceding claims, wherein the stimulation unit (120) comprises a plurality of stimulation elements (122; 124; 612; 614) configured to be arranged in relation to the part of the peripheral nervous system, wherein the stimulation elements (122; 124; 612; 614) comprise ultrasound transducers (122; 612) and/or electrodes (124; 614) for providing the selective stimulation of the part of the peripheral nervous system by an ultrasound stimulation signal and/or an electrical stimulation signal.

6. The apparatus according to any one of the preceding claims, wherein the sensor unit (110) and the stimulation unit (120) are arranged in a carrier (102) configured to conform to an outer surface of the part of the peripheral nervous system.

7. The apparatus according to claim 6, wherein the carrier (102) has form of a cuff configured to be arranged around a nerve (10).

8. The apparatus according to claim 6 or 7, wherein the sensor unit (110) comprises a set of ultrasound transducers and a set of electrodes, wherein the set of ultrasound transducers and the set of electrodes are arranged in a sensing area (130; 132) of the carrier (102) with the ultrasound transducers (112; 612) arranged intermixed with electrodes (114) such that at least one ultrasound transducer (112; 612) is arranged between two electrodes (114; 614).

9. The apparatus according to claim 8, wherein the sensor unit (110) comprises a first set and a second set of ultrasound transducers and a first set and a second set of electrodes, wherein the first set of ultrasound transducers and the first set of electrodes are arranged in a first sensing area (130) of the carrier (102) and the second set of ultrasound transducers and the second set of electrodes are arranged in a second sensing area (132) of the carrier (102), and wherein the first sensing area (130) and the second sensing area (132) are arranged on opposite sides of a stimulation area (134) of the carrier (102).

10. The apparatus according to any one of claims 6-9, wherein the carrier (102) comprises an acoustically transparent material (104) such that the carrier (102) is configured to be arranged in relation to the part of the peripheral nervous system with the acoustically transparent material (104) between the part of the peripheral nervous system and ultrasound transducers (112; 612).

11. The apparatus according to any one of the preceding claims, wherein the controller (140) is further configured to control one or more of a size of the location of the selective stimulation of the part of the peripheral nervous system, a stimulation modality of the stimulation signal, a type of effect by the stimulation signal on the part of the peripheral nervous system, and timing of the stimulation signal.

12. A system (200) for stimulating a part of a peripheral nervous system, said system (200) comprising:
the apparatus (100) according to any one of the preceding claims; and
a biomarker sensor (210) configured to sense information of a biomarker of a part of a body.

13. The system according to claim 12, said system (200) comprising a systemic controller (220) configured to receive the information of the biomarker and configured to monitor effect of the selective stimulation of the position within the part of the peripheral nervous system by the stimulation unit (120) of the apparatus (100).

14. The system according to claim 13, wherein the systemic controller (220) is configured to provide input to the controller (140) of the apparatus (100) based on the information of the biomarker for controlling the selective stimulation of the position within the part of the peripheral nervous system by the stimulation unit (120) of the apparatus (100).

15. A method for controlling stimulation of a part of a peripheral nervous system, said method comprising:
determining (302) structural information of the part of the peripheral nervous system;
determining (304) functional information of the part of the peripheral nervous system; and
determining (306) a location within the part of the peripheral nervous system for which selective stimulation is desired, wherein the location of the selective stimulation within the part of the peripheral nervous system is determined based on the structural information and the functional information.
